# EUROPÄISCHE PATENTSCHRIFT

(11) **EP 0 311 091 B1**
(45) Veröffentlichungstag und Bekanntmachung des Hinweises auf die Patenterteilung: **16.06.1993**
(21) Anmeldenummer: 88116623.5
(22) Anmeldetag: 07.10.1988
(51) Int. Cl.: A61K 31/20, A61K 31/23, A61K 9/10

(54) **Isotone omega-3-fettsäurenhaltige Fettemulsion und ihre Verwendung**
Isotonic lipid emulsions containing omega-3 fatty acids and the use thereof
Emulsions isotoniques de lipides contenant des acides gras oméga-3 et leur utilisation

(30) Priorität: 09.10.1987 DE 3734147
(43) Veröffentlichungstag der Anmeldung: 12.04.1989
(73) Patentinhaber: B. Braun Melsungen AG, 34209 Melsungen (DE)
(72) Erfinder: Nehne, Jörg, Dr., D-3502 Guxhagen (DE); Boll, Michael, Dr., D-3508 Melsungen (DE)
(74) Vertreter: von Kreisler, Alek, Dipl.-Chem.

(56) Entgegenhaltungen:
- EP-A- 0 115 419
- FR-A- 2 542 613
- GB-A- 2 033 745

## Beschreibung

Die vorliegende Erfindung betrifft isotone ω-3-fettsäurenhaltige Fettemulsionen zur parenteralen Applikation und ihre Verwendung im Postaggressionsstoffwechsel oder bei chronisch-entzündlichen Erkrankungen.

Fettemulsionen haben die Aufgabe, dem Organismus Fett in einer intravenös verträglichen Darreichungsform zuzuführen, wenn eine normale (enterale) Ernährung nicht möglich oder aus medizinischen Gründen kontraindiziert ist. Die mit den Fettemulsionen verabreichten mittelkettigen (MCT) oder langkettigen Triglyzeride (LCT) dienen dabei als Energiequelle und als Lieferant essentieller Fettsäuren, letzteres aber nur im Falle der langkettigen Triglyzeride und soweit diese mehrfach ungesättigte Fettsäuren enthalten.

Patienten in normalem Ernährungszustand verfügen meistens über ausreichende Fettreserven, um ihren Bedarf an Energie und essentiellen Fettsäuren bei kurzfristiger Nahrungskarenz aus eigenen Vorräten zu decken. Schwere und anhaltende Erkrankungen sind jedoch mit einem erhöhten Bedarf an essentiellen Fettsäuren verbunden, ebenso wie mit einem gesteigerten Energiebedarf und einer längeren Indikation zur parenteralen Ernährung. Sie stellen somit das Haupteinsatzgebiet für intravenöse Fettemulsionen dar. Kohlenhydratlösungen sind abgesehen davon, daß sie keine essentiellen Fettsäuren liefern, als alleinige Energiequelle bei schweren Erkrankungen ungeeignet, weil ihre Verwertung im Postaggressionsstoffwechsel gestört ist. Demgegenüber werden Fette, seien sie endogenen oder exogenen Ursprungs, im Postaggressionsstoffwechsel bevorzugt utilisiert. Diese Beobachtung unterstreicht die prinzipielle Eignung von Lipidemulsionen zur parenteralen Ernährung und zur Substitution von essentiellen Fettsäuren bei schweren Erkrankungen und den damit verbundenen Stoffwechselsituationen.

In qualitativer Hinsicht ist die bisherige Praxis der intravenösen Fettzufuhr allerdings unbefriedigend. Die zur Zeit verfügbaren Fettemulsionen werden aus Ölen pflanzlichen Ursprungs (Sojabohnenöl oder Safloröl) hergestellt; in einigen Fällen enthalten sie auch mittelkettige Triglyzeride.

Die Öle der Sojabohne und der Saflordistel sind durch einen hohen Anteil an mehrfach ungesättigen Fettsäuren der ω-6-Reihe gekennzeichnet (in der Hauptsache: Linolsäure, 18 : 2ω-6), während ihr Gehalt an ω-3-Fettsäuren gering ist. Im Sojabohnenöl beträgt der Anteil von ω-3- Fettsäuren am Gesamtfettsäurengehalt etwa 7 %, im Safloröl liegt er bei etwa 1 %. In beiden Fällen kommen die ω-3-Fettsäuren praktisch ausschließlich als α-Linolensäure (18 : 3 ω-3) vor. Mittelkettige Triglyzeride enthalten überhaupt keine ungesättigten Fettsäuren und somit weder ω-6- noch ω-3-Fettsäuren.

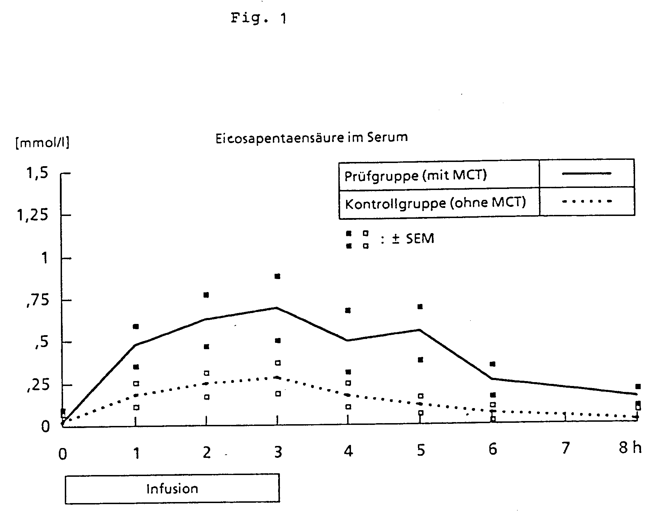

Die Einteilung von ungesättigten Fettsäuren in solche der ω-3- oder ω-6-Reihe basiert auf chemischen Strukturmerkmalen, genauer gesagt auf der Position der ungesättigten Bindungen im Fettsäurenmolekül. Der menschliche Organismus ist nicht in der Lage, vielfach ungesättigte Fettsäuren, die für ihn essentiell sind, d.h. auf deren Zufuhr mit der enteralen oder parenteralen Nahrung er angewiesen ist, selbst herzustellen. Er kann jedoch längerkettige ungesättigte Fettsäuren aus kürzerkettigen ungesättigten Fettsäuren synthetisieren, innerhalb der ω-6-Reihe z. B. Arachidonsäure (20 : 4 ω-6) aus Linolsäure und innerhalb der ω-3-Reihe Eikosapentaensäure (20 : 5 ω-3) aus α-Linolensäure (s. Abb. 1). Eine Bildung von ω-6-Fettsäuren aus Vorstufen der ω-3-Reihe oder umgekehrt ist nicht möglich. 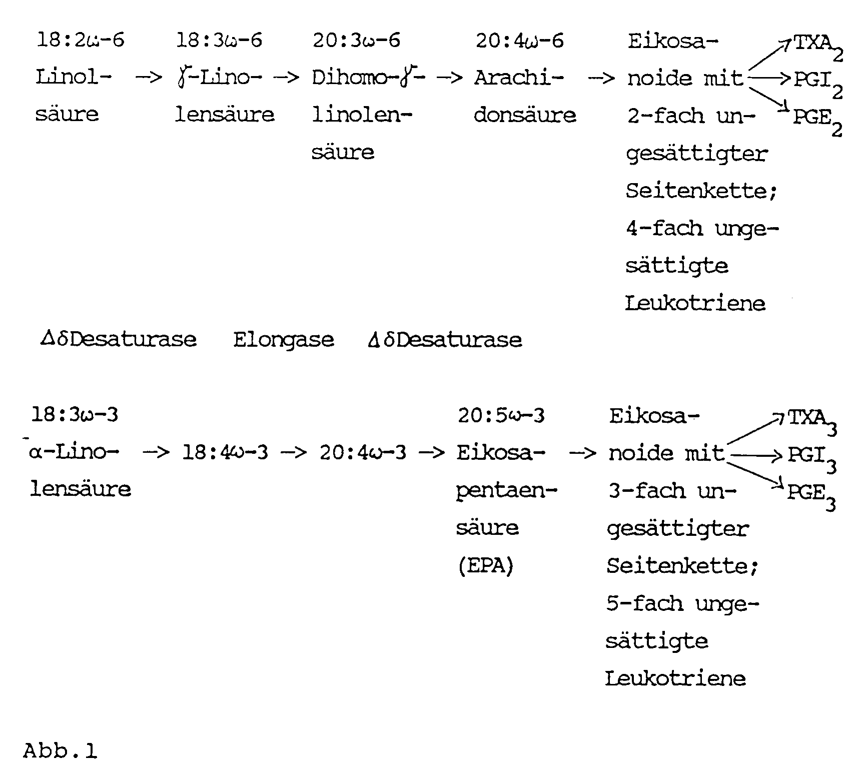
Sowohl ω-3- als auch ω-6-Fettsäuren sind für den Menschen essentiell, allerdings mit jeweils unterschiedlicher Wertigkeit.

Herkömmliche Fettemulsionen sind aufgrund des hohen Gehalts an Linolsäure gut geeignet, den Bedarf an ω-6-Fettsäuren zu decken. Anders sieht es bei den ω-3-Fettsäuren in den bisherigen Fettemulsionen aus: Hier ist der Gehalt an α-Linolensäure von vorneherein gering, und im Stoffwechsel konkurrieren Linolsäure und α-Linolensäure um die desaturierenden und elongierenden Enzyme. Deshalb findet eine Desaturierung und Elongation von α-Linolensäure in Gegenwart größerer Mengen Linolsäure, wie sie mit den bisherigen Fettemulsionen zugeführt wird, nur in äußerst geringem Umfang statt. Aus biologischer Sicht sind die ω-3-Fettsäuren trotz eines Anteils von 7 % an den Gesamtfettsäuren des Sojabohnenöls daher nahezu inaktiv. Die Bildung der Arachidonsäure, von der die biologischer Wirkungen der ω-6-Fettsäuren ausgehen, aus der im Sojabohnenöl oder Safloröl enthaltenen Linolsäure verläuft dagegen weitgehend ungestört.

Die biologische Bedeutung der essentiellen Fettsäuren besteht darin, daß sie Vorläufer von Prostaglandinen, Thromboxanen und Leukotrienen sind. Diese zusammenfassend als Eikosanoide bezeichneten Substanzen wirken ähnlich wie Hormone und beeinflussen zahlreiche Funktionen des Organismus wie Gefäßtonus, Gefäßpermeabilität, pulmonalen Gefäßwiderstand, Thrombusbildung, Blutdruckregulation, entzündlich-allergische Reaktionen u.a. Die Eikosanoide werden im Körper nahezu ubiquitär aus lokal freigesetzten Membranfettsäuren gebildet, Eikosanoide mit 3-fach ungesättigter Seitenkette aus Eikosapentaensäure und Eikosanoide mit 2-fach ungesättigter Seitenkette aus Arachidonsäure (s. Abb. 1). Je nach ihrer Herkunft aus einer dieser Fettsäurenfamilien haben die Eikosanoide unterschiedliche biologische Eigenschaften (s.u).

Da die Präkursor-Fettsäuren bzw. ihre Vorstufen (α-Linolensäure für die ω-3-Fettsäuren; Linolsäure für die ω-6-Fettsäuren) im menschlichen Organismus nicht selbst gebildet werden können, ist ihr Vorkommen im Körper von der Aufnahme mit der Nahrung oder - bei parenteraler Ernährung - von der intravenösen Zufuhr abhängig. In der sogenannten "westlichen Ernährung", wie sie in Europa und Amerika üblich ist, und - wie bereits ausgeführt - in den bisherigen Fettemulsionen zur parenteralen Ernährung überwiegen die ω-6-Fettsäuren bei weitem, und bei den eikosanoidvermittelten Wirkungen spielen folglich 2-fach ungesättigte Prostaglandine und Thromboxane die Hauptrolle. Anders bei traditionell von Fischkost lebenden Völkern wie Eskimos und Japanern. Ihre Nahrung enthält einen hohen Anteil an ω-3-Fettsäuren, den Präkursoren für 3-fach ungesättigte Eikosanoide.

Die biologischen Wirkungen von 3-fach ungesättigten Eikosanoiden, zu deren wichtigsten Vertretern PGI₃, PGE₃ und TXA₃ gehören, unterstützen z. T. die der 2-fach ungesättigten Eikosanoide (z.B. PGI₂, PGE₂ und TXA₂), z. T. hemmen sie diese. So wirkt PGI₃ ebenso stark gefäßerweiternd und aggregationshemmend wie PGI₂. Der gefäßkontrahierende und aggregationsfördernde Effekt des TXA₂ wird dagegen durch TXA₃, das selbst inaktiv ist, kompetitiv gehemmt. In ähnlicher Weise kann PGE₃ die bronchokonstriktorischen, ödemfördernden und immunsuppressiven Wirkungen von PGE₂ hemmen.

Ähnliche Interaktionen treten auch zwischen den Leukotrienen auf, die durch Lipoxygenierung aus der Arachidonsäure entstehen (z. B. LTB₄) und denen, die sich aus der Eikosapentaensäure ableiten (z. B. LTB₅). So ist LTB₄ ein hochpotenter Entzündungsmediator, während LTB₅ nur geringe Effektivität zeigt und infolgedessen die Wirkung von LTB₄ abschwächt.

Während also unter den 2-fach ungesättigten Eikosanoiden sowohl vasokonstriktorische, proaggregatorische als auch vasodilatatorische und antiaggregatorische Eigenschaften vorkommen, überwiegen bei den 3-fach ungesättigten Eikosanoiden gefäßerweiternde und aggregationshemmende Eigenschaften sowie Hemmwirkungen auf bestimmte 2-fach ungesättigte Eikosanoide. Bei Populationen mit hohem Verzehr von ω-3-fettsäurenreicher Nahrung wie den bereits erwähnten Eskimos oder Japanern hat dies im Vergleich zu Populationen mit "westlicher" Ernährung neben anderen Besonderheiten eine auffallend niedrige Rate arteriosklerotischer Gefäßerkrankungen zur Folge. Noch größere Bedeutung als bei "Stoffwechselgesunden" kommt den Prostaglandinwirkungen im Streßstoffwechsel zu: Normalerweise befinden sich die z. T. entgegengesetzten Wirkungen von 2-fach ungesättigten Eikosanoiden nämlich im Gleichgewicht. Unter dem Einfluß von Streßfaktoren wie Histamin, Bradykinin und Komplementfaktoren jedoch wird dieses Gleichgewicht auf die Seite der potentiell nachteiligen (vasokonstriktorischen, proaggregatorischen, proinflammatorischen) Eikosanoidwirkungen verschoben.

Diese Konstellation findet sich gehäuft bei schwerkranken Patienten und gerade solchen, die einer parenteralen Ernährung mit Fettemulsionen bedürfen: bei Patienten im Postaggressionsstoffwechsel nach Operationen und multiplen Traumen, bei septischen und beatmeten Patienten. Für solche Patienten ist das Vorherrschen von Streßfaktoren typisch, die das Gleichgewicht zugunsten der nachteiligen Wirkungen von 2-fach ungesättigten Eikosanoiden verschieben, und die unerwünschten Wirkungen werden durch die Verabreichung von Fettemulsionen mit hohem Linolsäuregehalt begünstigt. Auch bei schwerkranken Patienten, die keine Fettemulsionen im Rahmen der bei ihnen durchgeführten parenteralen Ernährung erhalten, sind nachteilige, durch 2-fach ungesättigte Eikosanoide vermittelte Wirkungen möglich. Aufgrund der vor der Erkrankung erfolgten Nahrungsaufnahme ist auch bei solchen Patienten, besonders nach vorausgegangener "westlicher" Ernährung, mit einem hohen Anteil von ω-6-Fettsäuren in den Membranfettsäuren zu rechnen und folglich auch mit den entsprechenden Eikosanoidwirkungen.

3-fach ungesättigte Eikosanoide, die die nachteilige Wirkung von 2-fach ungesättigten Eikosanoiden abschwächen, können bei den betreffenden Patienten meist nicht in dem erforderlichen Umfang gebildet werden, weil der Gehalt an ω-3-Fettsäuren in den Membranfettsäuren nach vorangegangener "westlicher" Ernährung zu gering und eine enterale Zufuhr von Eikosapentaensäure in dieser Situation nicht möglich ist. Die zur Überbrückung der Nahrungskarenz bisher verwendeten Fettemulsionen und ihre Komponenten enthalten entweder keine ω-3-Fettsäuren wie im Fall von Safloröl und mittelkettigen Triglyzeriden, oder die Bildung von Eikosapentaensäure aus α-Linolensäure wird durch den gleichzeitigen hohen Gehalt an Linolsäure inhibiert wie im Fall von Sojabohnenöl.

Experimentell konnte von J.W. Alexander, Arch. Surg. -Vol 121, Aug 1986, S 966 ff, gezeigt werden, daß Tiere mit einem Verbrennungstrauma (30 % der Körperoberfläche) Unter einer linolsäurereichen (ω-6-fettsäurenreichen) Diät eine signifikant kürzere Überlebenszeit, eine höhere metabolische Rate (entsprechend einem gesteigerten Streßstoffwechsel) und eine schlechtere Immunkompetenz besitzen als Tiere unter einer fischölreichen (ω-3-fettsäurenreichen) Vergleichsdiät. Durch Verabreichung von Indomethazin, eines Blockers der Prostaglandinsynthese, ließen sich die nachteiligen Wirkungen der linolsäurereichen Diät z.T. aufheben bzw. ähnlich günstige Resultate erzielen wie in der Fischölgruppe. Daraus geht hervor, daß Prostaglandine aus der ω-6-Fettsäuren- oder Linolsäure-Reihe unmittelbar für die schlechte Prognose der Tiere aus der betreffenden Gruppe verantwortlich sind.

In der klinischen Praxis ist die Hemmung der von der Arachidonsäure ausgehenden Prostaglandinsynthese durch Indomethazin allerdings problematisch, da hierbei nur Eikosanoide des Cyclooxygenasewegs blockiert werden, und dafür eine Verlagerung auf den Lipoxygenaseweg einsetzt, die mit einer verstärkten Ödembildung und einem erhöhten ARDS-Risiko verbunden ist wie von W. Seeger et al. und H. Wolf et al. in Klin. Wochenschr. 1981; 59: 459 ff bzw. 463 ff beschrieben.

Entsprechend dem bis hierher dargelegten Stand des Wissens besteht also Bedarf an einer Fettemulsion zur parenteralen Verabreichung im Postaggressionsstoffwechsel, die weniger Vorstufen für 2-fach ungesättigte Eikosanoide bzw. 4-fach ungesättigte Leukotriene, in der Praxis also weniger Linolsäure enthält, statt dessen jedoch Präkursoren für 3-fach ungesättigte Eikosanoide bzw. 5-fach ungesättigte Leukotriene, also ω-3-Fettsäuren, vorzugsweise Eikosapentaensäure. Ob die betreffende Fettemulsion nur ω-3-Fettsäuren oder eine Kombination von ω-3- und ω-6-Fettsäuren enthält, richtet sich danach, ob die akute Beeinflussung von Prostaglandinwirkungen durch ω-3-Fettsäuren oder ihre längerfristige Anwendung im Rahmen der parenteralen Ernährung im Vordergrund stehen. Im letzteren Fall ist auf ω-6-Fettsäuren (Linolsäure) wegen ihrer Essentialität nicht zu verzichten.

Von diesem Wissensstand ausgehend wurde daher vorgeschlagen, ω-3-Fettsäuren oder Ester derselben (z.B. ein gereinigtes Fischöl) allein oder zusammen mit einem Pflanzenöl zu emulgieren und in eine zur intravenösen Infusion oder sonstigen Anwendung geeignete Darreichungsform zu bringen.

Das US-Patent 4 526 902 beschreibt Mischungen aus 25 bis 75 Gew.-% Eikosapentaensäure und einer ω-6-Fettsäure, die enteral als Bestandteil von Pharmazeutika oder fetthaltigen Nahrungsmitteln wie Butter oder ähnlichem verwendet werden.

In der EP 0 120 169 B1 sind synthetische Triglyzeride beschrieben, die am mittleren C des Glyzerinmoleküls eine mehrfach ungesättigte Fettsäure, vorzugsweise Eikosapentaensäure, besitzen können. Die so erzeugten Glyzeride können als Nahrungsmittel, Nahrungsergänzungsmittel oder als Arzneimittel für die therapeutishge Ernährung verwendet werden.

EP-A-0 145 873 offenbart eine Transfusionsemulsion zur Nahrungsergänzung, die einen Fettanteil von 5 bis 20 w/v-%, Rest Wasser, enthält, und worin die Fettphase zu 10 bis 50 w/v-% aus α-Linolensäure oder ihrem Glyzerin- bzw. Ethylester und zu 50 bis 90 w/v-% aus einem ω-6-Fettsäure enthaltenden Pflanzenöl, wie Safloröl bzw. Sojaöl, besteht.

Die JP-OS Sho-58-230918 beschreibt eine Eikosapentaensäure enthaltende Emulsion zur oralen und nicht-oralen Verwendung. Diese enthält 1 bis 40 w/v-% Eikosapentaensäure bzw. vorzugsweise deren Methyl- oder Ethylester, 1 bis 30 w/v-% eines Pflanzenöls, vorzugsweise Sojaöl, 0,01 bis 30 w/v-% α-Tocopherol, und als Emulgatoren 0,1 bis 5 w/v-% eines Phospholipids, vorzugsweise aus Eigelb und/oder Soja, sowie 0,1 bis 10 w/v-% eines nichtionischen synthetischen Emulgators.

Die DE-OS 34 09 793 offenbart eine flüssige Emulsion zur Transfusion mit antithrombischer und antiarteriosklerotischer Wirkung, die zur Nahrungsergänzung dienen kann. Sie besteht neben Wasser zu 5 bis 20 w/v-% aus Eikosapentaensäure, Docosahexaensäure oder deren. Ester und sie ist vorzugsweise ein gereinigtes Fischöl, wie Sardinenöl. Weiterhin enthält sie 1 bis 19 w/v-% eines Pflanzenöls, vorzugsweise Soja- und/oder Safloröl, sowie 1 bis 2 w/v-% eines Phospholipidemulgators, vorzugsweise aus Eigelb oder Soja. Dieser Fettemulsion kann als Antioxidans α-Tocopherol zugefügt werden.

In den genannten Schriften blieb jedoch unberücksichtigt, daß unter diesen Bedingungen ω-3-Fettsäuren zum größten Teil im Energiestoffwechsel verwertet, also oxidiert werden. Dadurch werden sie anderen Aufgaben wie z.B. der Bildung von Eikosanoiden entzogen. Die Oxidation von Triglyzeriden zur Energiegewinnung ist im Postaggressionsstoffwechsel - wie bereits erwähnt - sogar besonders hoch. Das Verfahren der direkten "ungeschützten" Zufuhr von ω-3-fettsäurenhaltigen Fettemulsionen ist also zumindest unökonomisch - wenn überhaupt durchführbar, da erhebliche Zweifel an dem jeweils oxidierten bzw. zur Bildung von Eikosanoiden verfügbaren Anteil bestehen müssen. Mit anderen Worten kann von einer unter diesen Bedingungen gegebenen Dosis keine definierte Wirkung erwartet werden, da sich nicht absehen läßt, zu welchem Prozentsatz die ω-3-Fettsäuren anderweitig verwertet wurden.

Es ist demnach in Erweiterung der oben beschriebenen Aufgabe sicherzustellen, daß die zur Antagonisierung der unerwünschten Wirkungen 2-fach ungesättigter Eikosanoide und 4-fach ungesättigter Leukotriene verabreichten ω-3-Fettsäuren auch möglichst vollständig für diesen Zweck zur Verfügung stehen.

Überraschenderweise wurde nun gefunden, daß sich diese Aufgabe durch kombinierte Verabreichung von ω-3-Fettsäuren bzw. ω-3-fettsäurenhaltigem Fischöl oder einer Fischölfraktion mit mittelkettigen Triglyzeriden lösen läßt. Im einzelnen wurde nämlich gefunden, daß die Fettsäuren aus gereinigtem Fischöl, wenn dieses zusammen mit mittelkettigen Triglyzeriden zu einer Emulsion verarbeitet und intravenös infundiert wurde, langsamer aus dem Intravasalraum abströmten und in bedeutend geringerem Umfang oxidiert wurden als bei alleiniger Verabreichung einer Fischölemulsion.

Es wurde weiterhin gefunden, daß die kombinierte Verabreichung von ω-3-fettsäurenhaltigem, gereinigtem Fischöl bzw. einer Fischölfraktion mit mittelkettigen Triglyzeriden einen vorteilhaften leberprotektiven Effekt besitzt. So zeigten Ratten nach lägerfristiger Verabreichung einer Emulsion, die nur Triglyzeride aus Fischöl enthielt, ausgeprägte Fetteinlagerungen in der Leber, insbesondere auch in den Kupfferschen Sternzellen. Wurden die Ratten dagegen mit gleichen Menge von Triglyzeriden aus Fischöl, jedoch in gemeinsamer Emulsion mit mittelkettigen Triglyzeriden behandelt, fanden sich in der Leber praktisch keine Zeichen eine Fettinfiltration mehr.

Die Erfindung betrifft eine isotone Fettemulsion zur parenteralen Applikation, enthaltend ω-3-Fettsäuren, insbesondere Eikosapentaensäure (EPA) oder ihre physiologisch unbedenklichen Ester als Bestandteile der Fettphase, dadurch gekennzeichnet, daß die Fettemulsion
- diese ω-3-Fettsäuren oder Ester in Reinform oder als Bestandteil von Fischölen,
- mittelkettige Triglyzeride (MCT),
- mindestens einen physiologisch unbedenklichen Emulgator,
- gegebenfalls mindestens ein ω-6-Fettsäuren lieferndes Pflanzenöl,
- α-Tocopherol oder physiologisch unbedenkliche α-Tocopherolester sowie
- übliche Zusatz- und Hilfsstoffe enthält, wobei
- das Verhältnis von EPA oder ihren physiologisch unbedenklichen Estern zu MCT zwischen 1 : 9 und 3 : 5 liegt,
- der gesamte Fettgehalt zwischen 5 und 30 % liegt,
- der Emulgatorgehalt zwischen 5 und 12 % (bezogen auf den Fettgehalt) liegt und
- der Gehalt an ω-6-Fettsäuren lieferndem Pflanzenöl zwischen 0 und 30 % (bezogen auf den Fettgehalt) liegt.

ω-3-Fettsäuren, wie beispielsweise Docosahexaensäure, Eikosapentaensäure und deren Ester, insbesondere aber Eikosapentaensäure (EPA) oder ihre physiologisch unbedenklichen Ester können erfindungsgemäß entweder in Reinform oder als Bestandteil von Fischölen verwendet werden.

Geeignete Verbindungen sind neben der EPA ihre pharmakologisch unbedenklichen niederen Alkylester bzw. Glyzerinester. Bevorzugt sind der EPA-Ethylester bzw. der EPA-Glyzerinester.

Geeignete Fischöle sind beispielsweise solche, wie sie technisch in bedeutendem Umfang aus Kaltwasserfischen gewonnen werden. Bevorzugt sind hochgereinigte Fischölkonzentrate, die beispielsweise aus Makrele, Sardine, Hering oder Lachs gewonnen werden, wobei diese einen EPA-Gehalt von 20 bis 40 %, vorzugsweise mindestens 26 % (bezogen auf die Fettsäuremethylester des Fischölkonzentrats) besitzen.

Als mittelkettige Triglyzeride werden erfindungsgemäße solche verwendet, die zu mindestens 90 % aus Glyzeriden der Caprylsäure und Caprinsäure bestehen. Hierbei sollte das Verhältnis von EPA und ihren physiologisch unbedenklichen Estern zu mittelkettigen Triglyzeriden zwischen 1 : 9 und 3 : 5 liegen.

Als Emulgatoren werden physiologisch unbedenkliche Emulgatoren wie Phospholipide tierischen und pflanzlichen Ursprungs verwendet, insbesondere solche aus Hühnereigelb oder Soja.

Der Emulgatorgehalt beträgt 5 bis 12 % (bezogen auf den Fettgehalt) und der gesamte Fettgehalt der Fettemulsion beträgt zwischen 5 und 30 %.

Neben den hauptsächlich ω-3-Fettsäuren enthaltenden Komponenten kann als Eventualkomponente auch ein ω-6-Fettsäure lieferndes Pflanzenöl verwendet werden. Bevorzugt wird hiervon Safloröl und/oder Sojaöl verwendet, wobei der Gehalt an diesen Pflanzenölen zwischen 0 und 30 %, bezogen auf den Fettgehalt, liegt.

Als Emulgierhilfsstoffe können weiterhin Natriumsalze langkettiger Fettsäuren (bevorzugt in einer Konzentration von 0,005 bis 0,1 Gew.-%, bezogen auf die Gesamtemulsion) und/oder Cholesterin oder Cholesterinester (bevorzugt 0,005 bis 0,1 Gew.-%, bezogen auf die Gesamtemulsion) verwendet werden.

Die Fettemulsion weist vorzugsweise einen pH-Wert im Bereich von 6 bis 9 auf.

Schließlich kann erfindungsgemäß die Fettemulsion als Antioxidans und somit zum Schutz vor Peroxidbildung α-Tocopherol oder α-Tocopherol-Ester in einer Menge von 10 bis 1000 mg, bezogen auf 100 g Fett, enthalten, insbesondere wenn ω-3-Fettsäuren, insbesondere EPA und seine Ester in Reinform verwendet werden.

Die vorliegende Erfindung betrifft weiterhin ein Verfahren zur Herstellung der vorstehend beschriebenen Fettemulsionen.

Die Kombination von Fischöl mit mittelkettigen Triglyzeriden in der beschriebenen Weise hat eine verzögernde Wirkung auf die Clearance und Oxidation der Fettsäuren aus Fischöl zur Folge. Dadurch wird einerseits die Verweildauer der ω-3-Fettsäuren im Kreislauf erhöht und gleichsam ein Depoteffekt erzielt; zum anderen wird die nutritive (oxidative) Verwertung der ω-3-Fettsäuren zugunsten eines verstärkten Einbaus in Membranfettsäuren und Membranphospholipide sowie einer gesteigerten Verfügbarkeit für die Synthese von Eikosanoiden gebremst. Außerdem schützt die Kombination von Fischöl mit mittelkettigen Triglyzeriden die Leber vor der bei alleiniger Verabreichung von gereinigtem Fischöl, ω-3-fettsäurenhaltigen Fischölfraktionen oder ω-3-Fettsäurenestern zu beobachtenden fettigen Infiltration. Diese protektiven Wirkungen sind nicht vorhanden, wenn Fischöl mit Pflanzenöl statt mit MCT-Öl gemischt und in der beschriebenen Weise zu einer Emulsion verarbeitet wird. Die ω-3-fettsäurensparende und leberprotektive Wirkung ist also eines Folge der besonderen Kombination mit mittelkettigen Triglyzeriden.

Die Erfindung betrifft weiterhin eine Fettemulsion zur Verwendung im Postaggressionsstoffwechsel, insbesondere nach Operationen und multiplen Traumen, bei Verbrennungen, Infektionen, bei drohendem und manifestem Lungenversagen sowie zur Verwendung bei chronisch-entzündlichen Erkrankungen. Die erfindungsgemäße Fettemulsion kann ebenfalls in der Neonatologie und Pädiatrie verwendet werden.

Bei den Prozentangaben in der Beschreibung und in den Patentansprüchen handelt es sich jeweils, soweit nichts Anderes angegeben ist, um solche in Gramm pro 100 ml Lösung.

Die Erfindung wird durch folgende Beispiele erläutert.

### Beispiel 1

Eine Mischung aus 600 g hochgereinigtem Fischöl, 1400 g MCT, 2 g Cholesterinacetat, 120 g gereinigten Eiphospholipiden und 2 g Natriumstearat wird mittels eines Ultra-Turrax fein dispergiert. Mit Aqua ad iniectabilia, das 250 g Glycerol und 5 mmol NaOH enthält, wird unter Rühren auf 10 l aufgefüllt. Diese grobe Voremulsion wird in einem Hochdruckhomogenisator bei einem Druck von 400 kg/cm² homogenisiert. Nach Abfüllung in Glasflaschen geeigneter Qualität wird nach allgemein bekannten Verfahren hitzesterilisiert. Die Teilchen der sterilen und pyrogenfreien Emulsion sind kleiner 1 µm.

### Beispiel 2

Eine Mischung aus 400 g hochgereinigtem Fischöl, 600 g MCT, 90 g gereinigten Eiphospholipiden und 4 g Natriumstearat wird, wie unter 1 beschrieben, dispergiert und nach Auffüllen mit Aqua ad iniectabilia (enthält 250 g Glycerol) auf 10 l homogenisiert, abgefüllt und sterilisiert.

Die Emulsionstropfen sind kleiner 1 µm.

### Beispiel 3

Eine Mischung aus 300 g Eikosapentaensäureethylester, 700 g MCT, 60 g gereinigten Eiphospholipiden, 500 mg α-Tocopherol und 2 g Natriumstearat wird, wie unter 1 beschrieben, dispergiert und nach Auffüllen mit Aqua ad iniectabilia (enthält 125 g Glycerol) auf 5 l homogenisiert, abgefüllt und sterilisiert.

Die Emulsionstropfen sind kleiner 1 µm.

### Beispiel 4

Eine Mischung aus 300 g gereinigtem Fischöl, 600 g gereinigtem Safloröl, 1100 g MCT, 120 g gereinigten Eiphospholipiden, 200 mg α-Tocopherolacetat und 3,5 g Natriumstearat wird wie unter 1 beschrieben dispergiert. Nach Auffüllen auf 10 l mit Aqua ad iniectabilia, das 250 g Glycerol enthält, wird homogenisiert, abgefüllt und sterilisiert. Die Emulsionstropfen sind kleiner als 1 µm.

### Beispiel 5

Eine Mischung aus 200 g hochgereinigtem Fischöl, 300 g MCT und 90 g gereinigten Eiphospholipiden wird wie unter 1 beschrieben dispergiert und nach Auffüllen mit Aqua ad iniectabilia (enthält 250 g Glycerol) auf 10 l homogenisiert. Nach Einstellen des pH-Wertes mit NaOH auf pH 8,0 bis 8,5 wird abgefüllt und sterilisiert. Es resultiert eine Emulsion mit Fetttröpfchen kleiner als 1 µm.

### Beispiel 6

Eine Mischung aus 250 g hochgereinigtem Fischöl, 500 g MCT und 60 g gereinigten Sojaphospholipiden wird wie unter 1 beschrieben dispergiert und nach Auffüllen mit Aqua ad iniectabilia (enthält 125 g Glycerol) auf 5 l homogenisiert. Nach Einstellen des pH-Wertes mit NaOH auf pH 8,0 bis 8,5 wird abgefüllt und sterilisiert. Die Teilchen dieser Emulsion sind kleiner als 1 µm.

### Beispiel 7

100 g Eikosapentaensäure werden in 900 g MCT gelöst. Nach Zusatz von 90 g gereinigten Eiphospholipiden, 200 mg α-Tocopherol, 3 g Cholesterinacetat sowie 3 g Natriumstearat wird wie unter 1 beschrieben dispergiert und nach Auffüllen mit Aqua ad iniectabilia (enthält 250 g Glycerol) auf 10 l homogenisiert. Nach Einstellen des pH-Wertes mit NaOH auf pH 8,0 bis 8,5 wird abgefüllt und sterilisiert. Die resultierenden Emulsionstropfen sind kleiner als 1 µm.

### Beispiel 8

Eine Mischung aus 200 g Eikosapentaensäureethylester, 1600 g MCT, 200 gereinigtem Sojabohnenöl, 150 g gereinigten Eiphospholipiden, 500 mg α-Tocopherol und 4 g Natriumstearat wird wie in 1 beschrieben dispergiert und nach Auffüllen mit Aqua ad iniectabilia (enthält 250 g Glycerol) auf 10 l homogenisiert, abgefüllt und sterilisiert. Die Emulsionstropfen sind kleiner als 1 µm.

### Beispiel 9

Eine Mischung aus 150 g Eikosapentaensäureethylester, 1350 g MCT, 650 mg α-Tocopherol, 1,5 g Cholesterinacetat, 4 g Natriumstearat und 90 g gereinigten Eiphospholipiden wird wie unter 1 beschrieben dispergiert und nach Auffüllen auf 5 l mit Aqua ad iniectabilia (enthält 125 g Glycerol) homogenisiert, abgefüllt und sterilisiert. Die Teilchen der Emulsion sind kleiner als 1 µm.

### Beispiel 10

Eine Mischung aus 300 g hochgereinigtem Fischöl, 700 g MCT, 80 g gereinigten Sojaphospholipiden, 100 mg α-Tocopherolacetat und 3 g Natriumstearat wird wie unter 1 beschrieben dispergiert und nach Auffüllen auf 10 l mit Aqua ad iniectabilia (enthält 250 g Glycerol) homogenisiert, abgefüllt und sterilisiert. Die Emulsionströpfchen sind kleiner als 1 µm.

Die Wirkung der erfindungsgemäßen Emulsion wird durch folgende Beispiele erläutert:

### Anwendungsbeispiel 1 :

Bei Hunden wurde die Clearance von fischölhaltigen Emulsionen in Abhängigkeit von der Anwesenheit mittelkettiger Triglyceride in den Emulsionen untersucht. Zu diesem Zweck erhielten 6 Beagle-Hunde nach einem Übernachtfasten die intravenöse Infusion einer 10 %igen Emulsion aus hochgereinigtem Fischöl und MCT-Öl im Verhältnis 40 : 60 entsprechend dem Herstellungsbeispiel 2. Die Infusionsgeschwindigkeit betrug 1,5 ml/kg KG.h⁻¹ (≈ 60 mg Fischöl/kg KG.h⁻¹). Als Kontrolle wurde 3 Tage später bei den gleichen Tieren wiederum nach einem Übernachtfasten eine Emulsion mit 10 %igem alleinigem Fischöl-Gehalt mit einer Geschwindigkeit von 0,6 ml/kg KG.h⁻¹ (≈ 60 mg Fischöl/kg KG.h⁻¹) intravenös infundiert. Die Infusionsdauer betrug in beiden Fällen 3 Stunden. Während der Infusionen sowie einige Zeit danach wurden Blutproben entnommen und im Serum die Gesamtfettsäuren mittels Gaschromatographie analysiert.

Die Figur gibt den Konzentrationsverlauf der Eikosapentaensäure während und nach der Infusion in beiden Gruppen wieder.

Trotz der gleichen Dosis von Fischöl in beiden Gruppen stiegen die Serumkonzentrationen von Eikosapentaensäure in der Gruppe mit MCT-Gehalt in der Emulsion stärker und länger an.

### Anwendungsbeispiel 2

Mit Hilfe von ¹⁴C-markierter Eikosapentaensäure wurde die Oxidationsrate von Eikosapentaensäure bzw. Glycerol-tri-eikosapentaenoat in einem Tiermodell entsprechend W.J. Lossow und I.L. Chaikoff (Arch. Biochem. 57: 23-40, 1955) untersucht.

24 männliche Sprague-Dawley-Ratten (250 bis 300 g) wurden randomisiert in 4 Gruppen zu je 6 Tieren eingeteilt und erhielten Injektionen der folgenden Fettemulsionen:
- Gruppe I:: Fettemulsion (20 %) aus hochgereinigtem Fischöl
- Gruppe II:: Fettemulsion (20 %) aus hochgereinigtem Fischöl und Safloröl im Verhältnis 10 : 10 (w : w)
- Gruppe III:: Fettemulsion (20 %) entsprechend dem Herstellungsbeispiel 4 aus hochgereinigtem Fischöl, Safloröl und MCT-Öl im Verhältnis 3 : 3 : 14
- Gruppe IV:: Fettemulsion (20 %) entsprechend dem Herstellungsbeispiel l aus hochgereinigtem Fischöl und MCT-Öl im Verhältnis 6 : 14 (w : w).

In allen Emulsionen war der Fischöl-Anteil durch ¹⁴C-Glycerol-tri-eikosapentaenoat radioaktiv markiert. Die Aktivität der Emulsionen lag bei etwa 18 µCi/ml; die Fettdosis betrug 2 ml/kg KG (≈ 0,4 g/kg KG). Unmittelbar nach der Injektion wurden die Tiere in Stoffwechselkäfige eingebracht. Die Ausatemluft wurde über 6 h kontinuierlich abgesaugt und durch eine Oxifluor-Lösung geleitet. Die in der Oxifluor-Lösung als ¹⁴CO₂ zurückgehaltene Radioaktivität wurde im Szintillationszähler bestimmt und betrug in Prozent der insgesamt verabreichten Dosis:

| | |
|---|---|
| Gruppe I: | 42,8 ± 3,7 |
| Gruppe II: | 40,3 ± 3,9 |
| Gruppe III: | 17,2 ± 2,1^{*)} |
| Gruppe IV: | 16,5 ± 2,3^{*)} |

| | |
|---|---|
| ^{*)}: p < 0,001 (vs. I und II) | |

Die Oxidation von Eikosapentaensäure war in den Gruppen III und IV, d.h. bei der Infusion von Emulsionen mit gleichzeitigem MCT-Anteil, signifikant geringer als in den Gruppen I und II, bei denen die Fischölemulsionen keine mittelkettigen Trigylceride enthielten.

### Anwendungsbeispiel 3

18 männliche Wistar-Ratten (300 - 350 g) wurden in 3 Gruppen zu je 6 Tieren eingeteilt und an 20 aufeinanderfolgenden Tagen mit jeweils einer der nachstehenden Fettemulsionen infundiert.
- Gruppe I:: Fettemulsion (20 %) aus hochgereinigtem Fischöl Dosis: 22,5 ml/kg KG und Tag entspr. 4,5 g Fett/kg KG und Tag.
- Gruppe II:: Fettemulsion (20 %) aus hochgereinigtem Fischöl und Safloröl im Verhältnis 1 : 1 (w : w)
Dosis 45 ml/kg KG und Tag entspr. 9 g Fett/kg KG und Tag.
- Gruppe III:: Fettemulsion (20 %) aus hochgereinigtem Fischöl und MCT-Öl im Verhältnis 1 : 1 (w : w)
Dosis 45 ml/kg KG und Tag entspr. 9 g Fett/kg KG und Tag.

Die Infusionen erfolgten über einen in der Nackengegend der Tiere subkutan implantierten Katheter mit Einspritzport. Der Katheter wurde subkutan bis zur Vena jugularis geführt, in diese implantiert und bis in die Vena cava vorgeschoben. Die tägliche Infusionsdauer betrug 7 Stunden. 36 Stunden nach der letzten Infusion wurden die Tiere in Ethernarkose durch Entbluten getötet und unmittelbar danach seziert. Herz, Leber, Milz, Thymus, Nieren und Hoden wurden entnommen, gewogen und histologisch untersucht.

In der Gruppe II verstarb ein Tier am 16. Infusionstag; in den Gruppen I und III überlebten alle Tiere.

In der nachstehenden Übersicht beziehen sich die Werte auf die Organgewichte in g (Mittelwerte +/- SD bezogen auf ein Körpergewicht von 1000 g).

| | Herz | Leber | Milz | Nieren | Hoden | Thymus |
|---|---|---|---|---|---|---|
| Gruppe I | 3,2+/-0,2 | 43,3+/-1,6 | 3,6+/-0,4 | 4,8+/-0,5 | 10,5+/-0,8 | 1,4+/-0,2 |
| Gruppe II | 3,5+/-0,3 | 46,3+/-2,2 | 3,8+/-0,3 | 5,2+/-0,3 | 11,7+/-0,6 | 1,6+/-0,4 |
| Gruppe III | 3,3+/-0,2 | 38,6+/-1,5*) | 2,0+/-0,3*) | 5,1+/-0,3 | 11,2+/-0,5 | 1,3+/-0,2 |

| | | | | | | |
|---|---|---|---|---|---|---|
| *) p < 0,05: Gruppe III vs. Gruppe I bzw. II | | | | | | |

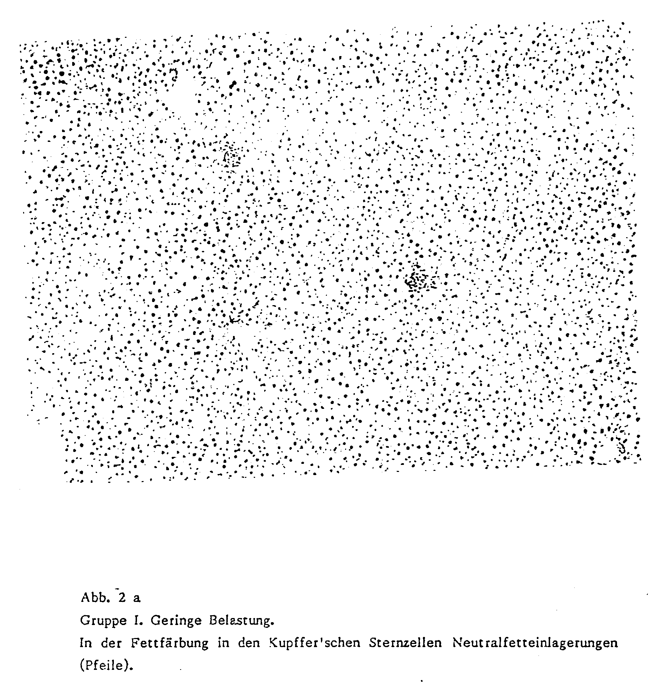

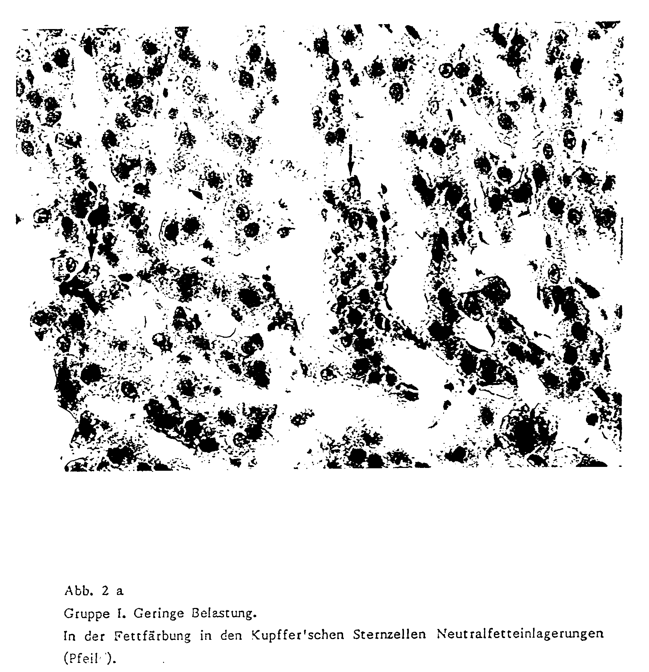

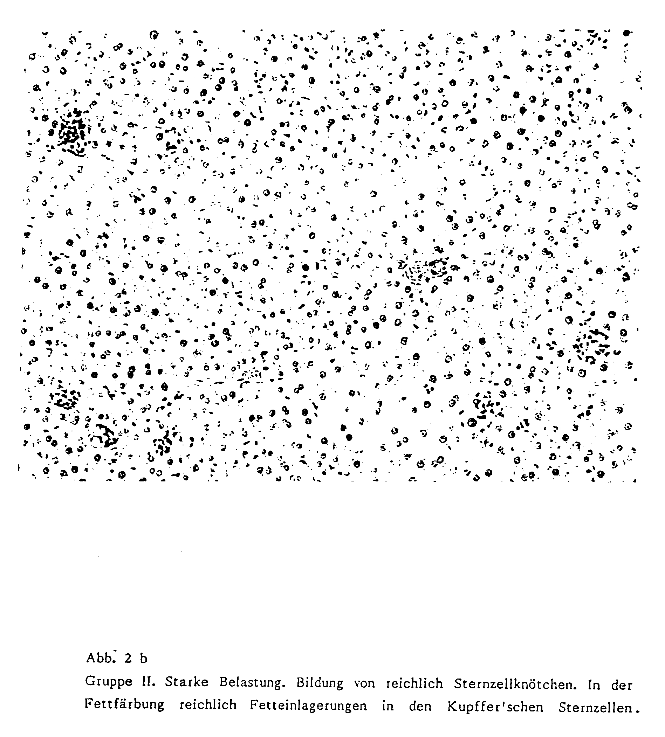

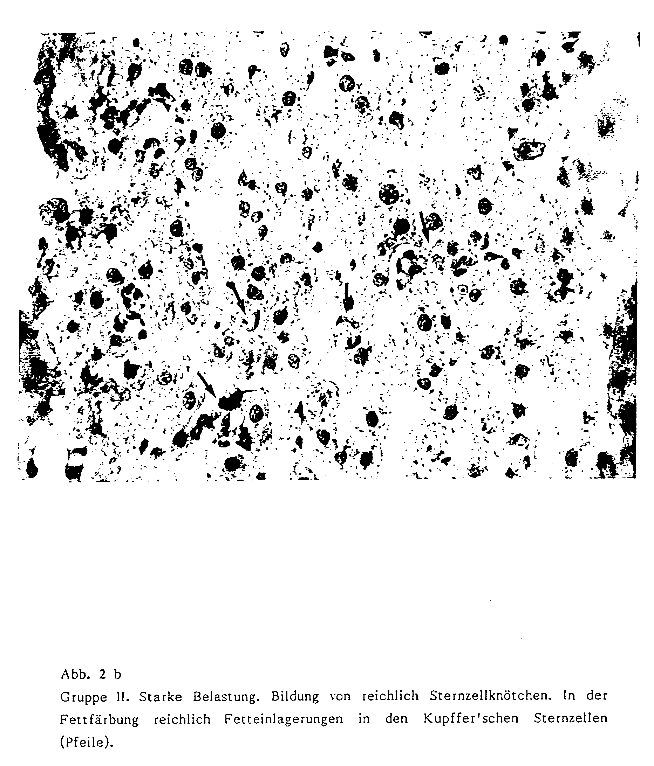

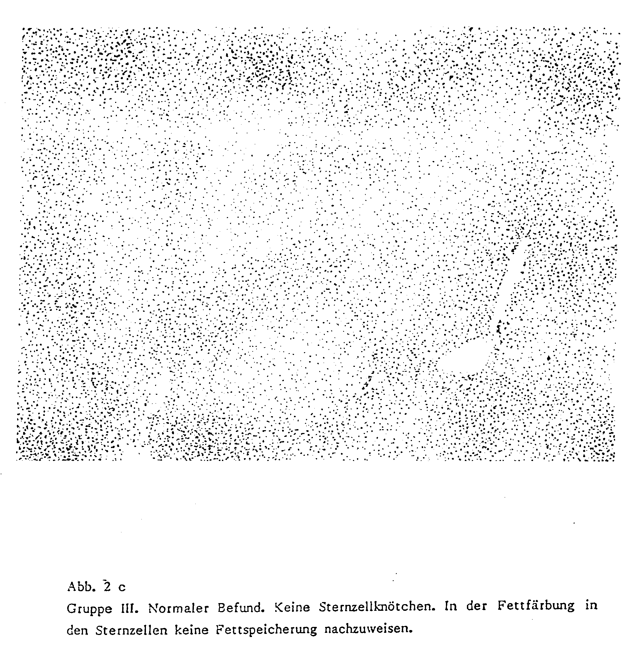

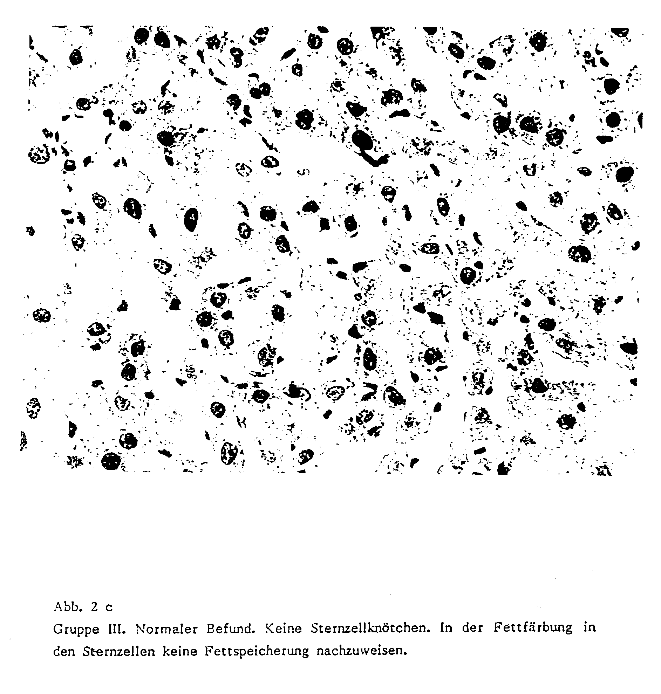

In der Gruppe III, in der eine Emulsion aus Fischöl und MCT-Öl verabreicht worden war, zeigten sich gemäß vorstehender Übersicht signifikant geringere Gewichte von Leber und Milz als in den Gruppen I (alleinige Zufuhr einer Fischölemulsion) und II (Fischöl mit Safloröl zur gemeinsamen Emulsion verarbeitet). Dem geringeren Lebergewicht in Gruppe III entsprach histologisch ein normaler Leberbefund, während in den Gruppen I und II eine Speicherung von Neutralfett vor allem in den Kupffer'schen Sternzellen der Leber mit Ausbildung von Sternzellknötchen sowie eine Speicherung von Neutralfett in den Parenchymzellen nachweisbar war, wie sich aus Figur 2 ergibt.

## Patentansprüche (Patentansprüche für folgende(n) Vertragsstaat(en): AT, BE, CH, DE, FR, GB, IT, LI, LU, NL, SE)

1. Isotone Fettemulsion zur parenteralen Applikation, enthaltend ω-3-Fettsäuren, insbesondere Eikosapentaensäure (EPA) oder ihre physiologisch unbedenklichen Ester als Bestandteile der Fettphase, dadurch gekennzeichnet, daß die die Fettemulsion
- diese ω-3-Fettsäuren oder Ester in Reinform oder als Bestandteil von Fischölen,
- mittelkettige Triglyzeride (MCT),
- mindestens einen physiologisch unbedenklichen Emulgator,
- gegebenenfalls mindestens ein ω-6-Fettsäuren lieferndes Pflanzenöl,
- α-Tocopherol oder physiologisch unbedenkliche α-Tocopherolester, sowie
- übliche Zusatz- und Hilfsstoffe
enthält, wobei
- das Verhältnis von EPA oder ihren physiologisch unbedenklichen Estern zu MCT zwischen 1 : 9 und 3 : 5 liegt,
- der gesamte Fettgehalt zwischen 5 und 30 % liegt,
- der Emulgatorgehalt zwischen 5 und 12 % (bezogen auf den Fettgehalt) liegt und
- der Gehalt an ω-6-Fettsäuren liefernden Pflanzenölen zwischen 0 und 30 % (bezogen auf den Fettgehalt) liegt.

2. Fettemulsion gemäß Anspruch 1, dadurch gekennzeichnet, daß dieses Fischöl erhältlich ist durch Verarbeitung von Kaltwasserfischen, beispielsweise von Makrele, Sardine, Hering oder Lachs.

3. Fettemulsion gemäß Anspruch 1 oder 2, dadurch gekennzeichnet, daß das Fischöl ein hochgereinigtes Fischölkonzentrat mit einem Gehalt an EPA von 20 bis 40 %, vorzugsweise mindestens 26 % (bezogen auf die Fettsäuremethylester des Fischölkonzentrats) ist.

4. Fettemulsion gemäß Ansprüchen 1 bis 3, dadurch gekennzeichnet, daß die mittelkettigen Triglyceride zu mindestens 90 % aus Glyzeriden der Caprylsäure (C₈) und Caprinsäure (C₁₀) bestehen.

5. Fettemulsion gemäß Ansprüchen 1 bis 4, dadurch gekennzeichnet, daß der eingesetzte Emulgator ein Phospholipid tierischen oder pflanzlichen Ursprungs, insbesondere aus Hühnereigelb oder Sojabohne, ist.

6. Fettemulsion gemäß Ansprüchen 1 bis 5, dadurch gekennzeichnet, daß sie weitere Emulgierhilfsstoffe wie Natriumsalze langkettiger Fettsäuren (bevorzugt 0,005 bis 0,1 Gew.-%, bezogen auf die Gesamtemulsion) und/oder Cholesterin oder Cholesterinester (bevorzugt 0,005 bis 0,1 Gew.-%, bezogen auf die Gesamtemulsion enthält.

7. Fettemulsion gemäß Ansprüchen 1 bis 6, dadurch gekennzeichnet, daß man als ω-6-Fettsäuren lieferndes Pflanzenöl Saflor- und/oder Sojaöl verwendet.

8. Fettemulsion gemäß Ansprüchen 1 bis 7, dadurch gekennzeichnet, daß sie 10 bis 1000 mg α-Tocopherol oder α-Tocopherolester, bezogen auf 100 g Fett, enthält.

9. Fettemulsion gemäß Ansprüchen 1 bis 8, dadurch gekennzeichnet, daß sie einen pH-Wert im Bereich von 6 bis 9 aufweist.

10. Fettemulsion gemäß Ansprüchen 1 bis 9 zur Verwendung im Postaggressionsstoffwechsel, insbesondere nach Operationen und multiplen Traumen, bei Verbrennungen, Infektionen, bei drohendem und manifestem Lungenversagen.

11. Fettemulsion gemäß Ansprüchen 1 bis 9 zur Verwendung bei chronisch-entzündlichen Erkrankungen, insbesondere bei chronisch-entzündlichen Darmerkrankungen.

12. Fettemulsion gemäß Ansprüchen 1 bis 9 zur Verwendung in der Neonatologie und Pädiatrie.

13. Verfahren zur Herstellung einer isotonen Fettemulsion zur parenteralen Applikation, enthaltend ω-3-Fettsäuren, insbesondere Eikosapentaensäure (EPA) oder ihre physiologisch unbedenklichen Ester als Bestandteile der Fettphase, dadurch gekennzeichnet, daß man diese Fettemulsion, welche
- diese ω-3-Fettsäuren oder Ester in Reinform oder als Bestandteil von Fischölen,
- mittelkettige Triglyzeride (MCT),
- mindestens einen physiologisch unbedenklichen Emulgator,
- gegebenenfalls mindestens ein ω-6-Fettsäuren lieferndes Pflanzenöl,
- α-Tocopherol oder physiologisch unbedenkliche α-Tocopherolester, sowie
- übliche Zusatz- und Hilfsstoffe
enthält, wobei
- das Verhältnis von EPA oder ihren physiologisch unbedenklichen Estern zu MCT zwischen 1 : 9 und 3 : 5 liegt,
- der gesamte Fettgehalt zwischen 5 und 30 % liegt,
- der Emulgatorgehalt zwischen 5 und 12 % (bezogen auf den Fettgehalt) liegt und
- der Gehalt an ω-6-Fettsäuren liefernden Pflanzenölen zwischen 0 und 30 % (bezogen auf den Fettgehalt) liegt,
durch Vermischen der Bestandteile erhält.

14. Verfahren gemäß Anspruch 13, dadurch gekennzeichnet, daß man das dort verwendete Fischöl durch Verarbeitung von Kaltwasserfischen, beispielsweise von Makrele, Sardine, Hering oder Lachs erhält.

15. Verfahren gemäß Anspruch 13 oder 14, dadurch gekennzeichnet, daß als Fischöl ein hochgereinigtes Fischölkonzentrat mit einem Gehalt an EPA von 20 bis 40 %, vorzugsweise mindestens 26 % (bezogen auf die Fettsäuremethylester des Fischölkonzentrats) eingesetzt wird.

16. Verfahren gemäß Ansprüchen 13 bis 15, dadurch gekennzeichnet, daß als mittelkettige Triglyceride solche Stoffe eingesetzt werden, die zu mindestens 90 % aus Glyzeriden der Caprylsäure (C₈) und Caprinsäure (C₁₀) bestehen.

17. Verfahren gemäß Ansprüchen 13 bis 16, dadurch gekennzeichnet, daß als Emulgator ein Phospholipid tierischen oder pflanzlichen Ursprungs, insbesondere aus Hühnereigelb oder Sojabohne, eingesetzt wird.

18. Verfahren gemäß Ansprüchen 13 bis 17, dadurch gekennzeichnet, daß als weitere Emulgierhilfsstoffe Natriumsalze langkettiger Fettsäuren (bevorzugt 0,005 bis 0,1 Gew.-%, bezogen auf die Gesamtemulsion) und/oder Cholesterin oder Cholesterinester (bevorzugt 0,005 bis 0,1 Gew.-%, bezogen auf die Gesamtemulsion) eingesetzt werden.

19. Verfahren gemäß Ansprüchen 13 bis 18, dadurch gekennzeichnet, daß man als ω-6-Fettsäuren lieferndes Pflanzenöl Saflor- und/oder Sojaöl verwendet.

20. Verfahren gemäß Ansprüchen 13 bis 19, dadurch gekennzeichnet, daß man 10 bis 100 mg α-Tocopherol oder α-Tocopherolester, bezogen auf 100 g Fett, verwendet.

21. Verfahren gemäß Ansprüchen 13 bis 20, dadurch gekennzeichnet, daß man die Fettemulsion auf einen pH-Wert im Bereich von 6 bis 9 einstellt.

22. Verfahren gemäß Ansprüchen 13 bis 21 zur Verwendung der hierbei erhaltenen Fettemulsion im Postaggressionsstoffwechsel, insbesondere nach Operationen und multiplen Traumen, bei Verbrennungen, Infektionen, bei drohendem und manifestem Lungenversagen.

23. Verfahren gemäß Ansprüchen 13 bis 21 zur Verwendung der hierbei erhaltenen Fettemulsion bei chronisch-entzündlichen Erkrankungen, insbesondere bei chronisch-entzündlichen Darmerkrankungen.

24. Verfahren gemäß Ansprüchen 13 bis 21 zur Verwendung der hierbei erhaltenen Fettemulsion in der Neonatologie und Pädiatrie.

## Patentansprüche (Patentansprüche für folgende(n) Vertragsstaat(en): ES, GR)

1. Verfahren zur Herstellung einer isotonen Fettemulsion zur parenteralen Applikation, enthaltend ω-3-Fettsäuren, insbesondere Eikosapentaensäure (EPA) oder ihre physiologisch unbedenklichen Ester als Bestandteile der Fettphase, dadurch gekennzeichnet, daß man diese Fettemulsion, welche
- diese ω-3-Fettsäuren oder Ester in Reinform oder als Bestandteil von Fischölen,
- mittelkettige Triglyzeride (MCT),
- mindestens einen physiologisch unbedenklichen Emulgator,
- gegebenenfalls mindestens ein ω-6-Fettsäuren lieferndes Pflanzenöl,
- α-Tocopherol oder physiologisch unbedenkliche α-Tocopherolester, sowie
- übliche Zusatz- und Hilfsstoffe
enthält, wobei
- das Verhältnis von EPA oder ihren physiologisch unbedenklichen Estern zu MCT zwischen 1 : 9 und 3 : 5 liegt,
- der gesamte Fettgehalt zwischen 5 und 30 % liegt,
- der Emulgatorgehalt zwischen 5 und 12 % (bezogen auf den Fettgehalt) liegt und
- der Gehalt an ω-6-Fettsäuren liefernden Pflanzenölen zwischen 0 und 30 % (bezogen auf den Fettgehalt) liegt,
durch Vermischen der Bestandteile erhält.

2. Verfahren gemäß Anspruch 1, dadurch gekennzeichnet, daß man das dort verwendete Fischöl durch Verarbeitung von Kaltwasserfischen, beispielsweise von Makrele, Sardine, Hering oder Lachs erhält.

3. Verfahren gemäß Anspruch 1 oder 2, dadurch gekennzeichnet, daß als Fischöl ein hochgereinigtes Fischölkonzentrat mit einem Gehalt an EPA von 20 bis 40 %, vorzugsweise mindestens 26 % (bezogen auf die Fettsäuremethylester des Fischölkonzentrats) eingesetzt wird.

4. Verfahren gemäß Ansprüchen 1 bis 3, dadurch gekennzeichnet, daß als mittelkettige Triglyceride solche Stoffe eingesetzt werden, die zu mindestens 90 % aus Glyzeriden der Caprylsäure (C₈) und Caprinsäure (C₁₀) bestehen.

5. Verfahren gemäß Ansprüchen 1 bis 4, dadurch gekennzeichnet, daß als Emulgator ein Phospholipid tierischen oder pflanzlichen Ursprungs, insbesondere aus Hühnereigelb oder Sojabohne, eingesetzt wird.

6. Verfahren gemäß Ansprüchen 1 bis 5, dadurch gekennzeichnet, daß als weitere Emulgierhilfsstoffe Natriumsalze langkettiger Fettsäuren (bevorzugt 0,005 bis 0,1 Gew.-%, bezogen auf die Gesamtemulsion) und/oder Cholesterin oder Cholesterinester (bevorzugt 0,005 bis 0,1 Gew.-%, bezogen auf die Gesamtemulsion) eingesetzt werden.

7. Verfahren gemäß Ansprüchen 1 bis 6, dadurch gekennzeichnet, daß man als ω-6-Fettsäuren lieferndes Pflanzenöl Saflor- und/oder Sojaöl verwendet.

8. Verfahren gemäß Ansprüchen 1 bis 7, dadurch gekennzeichnet, daß man 10 bis 100 mg α-Tocopherol oder α-Tocopherolester, bezogen auf 100 g Fett, verwendet.

9. Verfahren gemäß Ansprüchen 1 bis 8, dadurch gekennzeichnet, daß man die Fettemulsion auf einen pH-Wert im Bereich von 6 bis 9 einstellt.

10. Verfahren gemäß Ansprüchen 1 bis 9 zur Verwendung der hierbei erhaltenen Fettemulsion im Postaggressionsstoffwechsel, insbesondere nach Operationen und multiplen Traumen, bei Verbrennungen, Infektionen, bei drohendem und manifestem Lungenversagen.

11. Verfahren gemäß Ansprüchen 1 bis 10 zur Verwendung der hierbei erhaltenen Fettemulsion bei chronischentzündlichen Erkrankungen, insbesondere bei chronisch-entzündlichen Darmerkrankungen.

12. Verfahren gemäß Ansprüchen 1 bis 10 zur Verwendung der hierbei erhaltenen Fettemulsion in der Neonatologie und Pädiatrie.

## Claims (Claims for the following Contracting State(s): AT, BE, CH, DE, FR, GB, IT, LI, LU, NL, SE)

1. An isotonic fat emulsion for parenteral application, containing ω-3-fatty acids, and more specifically eicosapentaenoic acid (EPA) or its physiologically acceptable esters as components of the fat phase, characterized in that the fat emulsion contains
- these ω-3-fatty acids or esters in the pure state or as component(s) of fish oils,
- medium-chain triglycerides (MCT),
- at least one physiologically acceptable emulsifier,
- optionally at least one vegetable oil providing ω-6-fatty acids,
- α-tocopherol or physiologically acceptable α-tocopherol esters,
- and conventional additives or auxiliary materials,
whereby
- the ratio of EPA or its physiologically acceptable esters and MCT is between 1:9 and 3:5,
- the total fat contents is between 5 and 30%,
- the emulsifier contents is between 5 and 12% (based on the fat contents), and
- the contents of vegetable oil providing ω-6-fatty acids is between 0 and 30% (based on the fat contents).

2. The fat emulsion according to claim 1, characterized in that said fish oil is obtainable by processing cold water fish such as, for example, mackerel, sardine, herring or salmon.

3. The fat emulsion according to claims 1 or 2, characterized in that said fish oil is a highly purified fish oil concentrate having an EPA content of from 20 to 40%, and preferably of at least 26% (based on the fatty acid methyl esters of the fish oil concentrate).

4. The fat emulsion according to claims 1 to 3, characterized in that at least 90% of the medium-chain triglycerides employed consist of glycerides of caprylic (C₈) and capric (C₁₀) acids.

5. The fat emulsion according to claims 1 to 4, characterized in that the emulsifier employed is a phospholipid of animal or vegetable origin, and more particularly one derived from hen's egg yolk or soybean.

6. The fat emulsion according to claims 1 to 5, characterized in that it contains further emulsifying auxiliary materials such as sodium salts of long-chain fatty acids (preferably in a concentration of from 0.005 to 0.1% by weight, based on the total emulsion) and/or cholesterol or cholesterol esters (preferably in a concentration of from 0.005 to 0.1% by weight, based on the total emulsion).

7. The fat emulsion according to claims 1 to 6, characterized in that it contains safflower oil and/or soybean oil as the vegetable oil providing ω-6-fatty acids.

8. The fat emulsion according to claims 1 to 7, characterized in that it contains α-tocopherol or α-tocopherol ester in an amount of from 10 to 1000 mg, based on 100 g of fat.

9. The fat emulsion according to claims 1 to 8, characterized in that it has a pH value within the range of from 6 to 9.

10. The fat emulsion according to claims 1 to 9 for use in post-aggression metabolism, and more specifically after surgery and multiple traumata, in cases of burns, infections and of pending and manifest lung failure.

11. The fat emulsion according to claims 1 to 9 for use in the treatment of chronic inflammatory diseases, and more specifically, of chronic inflammatory intestinal diseases.

12. The fat emulsion according to claims 1 to 9 for use in neonatology and paediatrics.

13. A process for preparing an isotonic fat emulsion for parenteral application, containing ω-3-fatty acids, and more specifically eicosapentaenoic acid (EPA) or its physiologically acceptable esters as components of the fat phase, characterized in that said fat emulsion, which contains
- these ω-3-fatty acids or esters in the pure state or as component(s) of fish oils,
- medium-chain triglycerides (MCT),
- at least one physiologically acceptable emulsifier,
- optionally at least one vegetable oil providing ω-6-fatty acids,
- α-tocopherol or physiologically acceptable α-tocopherol esters,
- and conventional additives or auxiliary materials,
whereby
- the ratio of EPA or its physiologically acceptable esters and MCT is between 1:9 and 3:5,
- the total fat contents is between 5 and 30%,
- the emulsifier contents is between 5 and 12% (based on the fat contents), and
- the contents of vegetable oil providing ω-6-fatty acids is between 0 and 30% (based on the fat contents),
is obtained by mixing the components.

14. The process according to claim 13, characterized in that the fish oil used therein is obtained by processing cold water fish such as, for example, mackerel, sardine, herring or salmon.

15. The process according to claims 13 and 14, characterized in that as the fish oil there is employed a highly purified fish oil concentrate having an EPA content of from 20 to 40%, and preferably of at least 26% (based on the fatty acid methyl esters of the fish oil concentrate).

16. The process according to claims 13 to 15, characterized in that as the medium-chain triglycerides there are employed materials at least 90% of which consist of glycerides of caprylic (C₈) and capric (C₁₀) acids.

17. The process according to claims 13 to 16, characterized in that the as the emulsifier there is employed a phospholipid of animal or vegetable origin, and more particularly one derived from hen's egg yolk or soybean.

18. The process according to claims 13 to 17, characterized in that as further emulsifying auxiliary materials there are employed sodium salts of long-chain fatty acids (preferably in a concentration of from 0.005 to 0.1% by weight, based on the total emulsion) and/or cholesterol or cholesterol esters (preferably in a concentration of from 0.005 to 0.1% by weight, based on the total emulsion).

19. The process according to claims 13 to 18, characterized in that safflower oil and/or soybean oil is employed as the vegetable oil providing ω-6-fatty acids.

20. The process according to claims 13 to 19, characterized in that α-tocopherol or α-tocopherol ester is used in an amount of from 10 to 1000 mg, based on 100 g of fat.

21. The process according to claims 13 to 20, characterized in that the fat emulsion is adjusted to a pH value within the range of from 6 to 9.

22. The process according to claims 13 to 21 for use of the fat emulsion obtained thereby in post-aggression metabolism, and more specifically after surgery and multiple traumata, in cases of burns, infections and of pending and manifest lung failure.

23. The process according to claims 13 to 21 for use of the fat emulsion obtained thereby in the treatment of chronic inflammatory diseases, and more specifically, of chronic inflammatory intestinal diseases.

24. The process according to claims 13 to 21 for use of the fat emulsion obtained thereby in neonatology and paediatrics.

## Claims (Claims for the following Contracting State(s): ES, GR)

1. A process for preparing an isotonic fat emulsion for parenteral application, containing ω-3-fatty acids, and more specifically eicosapentaenoic acid (EPA) or its physiologically acceptable esters as components of the fat phase, characterized in that said fat emulsion, which contains
- these ω-3-fatty acids or esters in the pure state or as component(s) of fish oils,
- medium-chain triglycerides (MCT),
- at least one physiologically acceptable emulsifier,
- optionally at least one vegetable oil providing ω-6-fatty acids,
- α-tocopherol or physiologically acceptable α-tocopherol esters,
- and conventional additives or auxiliary materials,
whereby
- the ratio of EPA or its physiologically acceptable esters and MCT is between 1:9 and 3:5,
- the total fat contents is between 5 and 30%,
- the emulsifier contents is between 5 and 12% (based on the fat contents), and
- the contents of vegetable oil providing ω-6-fatty acids is between 0 and 30% (based on the fat contents),
is obtained by mixing the components.

2. The process according to claim 1, characterized in that the fish oil used therein is obtained by processing cold water fish such as, for example, mackerel, sardine, herring or salmon.

3. The process according to claims 1 and 2, characterized in that as the fish oil there is employed a highly purified fish oil concentrate having an EPA content of from 20 to 40%, and preferably of at least 26% (based on the fatty acid methyl esters of the fish oil concentrate).

4. The process according to claims 1 to 3, characterized in that as the medium-chain triglycerides there are employed materials at least 90% of which consist of glycerides of caprylic (C₈) and capric (C₁₀) acids.

5. The process according to claims 1 to 4, characterized in that the as the emulsifier there is employed a phospholipid of animal or vegetable origin, and more particularly one derived from hen's egg yolk or soybean.

6. The process according to claims 1 to 5, characterized in that as further emulsifying auxiliary materials there are employed sodium salts of long-chain fatty acids (preferably in a concentration of from 0.005 to 0.1% by weight, based on the total emulsion) and/or cholesterol or cholesterol esters (preferably in a concentration of from 0.005 to 0.1% by weight, based on the total emulsion).

7. The process according to claims 1 to 6, characterized in that safflower oil and/or soybean oil is employed as the vegetable oil providing ω-6-fatty acids.

8. The process according to claims 1 to 7, characterized in that α-tocopherol or α-tocopherol ester is used in an amount of from 10 to 1000 mg, based on 100 g of fat.

9. The process according to claims 1 to 8, characterized in that the fat emulsion is adjusted to a pH value within the range of from 6 to 9.

10. The process according to claims 1 to 9 for use of the fat emulsion obtained thereby in post-aggression metabolism, and more specifically after surgery and multiple traumata, in cases of burns, infections and of pending and manifest lung failure.

11. The process according to claims 1 to 10 for use of the fat emulsion obtained thereby in the treatment of chronic inflammatory diseases, and more specifically, of chronic inflammatory intestinal diseases.

12. The process according to claims 1 to 11 for use of the fat emulsion obtained thereby in neonatology and paediatrics.

## Revendications (Revendications pour l'(les) Etat(s) contractant(s) suivant(s): AT, BE, CH, DE, FR, GB, IT, LI, LU, NL, SE)

1. Emulsion grasse isotonique pour administration parentérale, contenant des acides gras en oméga-3, notamment de l'acide éicosapentaénoïque (EPA) ou leurs esters physiologiquement inoffensifs en tant que constituants de la phase grasse, caractérisée en ce que l'émulsion grasse contient :
- ces acides gras en oméga-3 ou ces esters sous forme pure ou en tant que constituants d'huiles de poisson,
- des triglycérides à longueur moyenne de chaîne (MCT),
- au moins un émulsifiant physiologiquement inoffensif,
- éventuellement au moins une huile végétale donnant des acides gras en oméga-6,
- de l'alpha-tocophérol ou un ester d'alpha-tocophérol physiologiquement inoffensif, ainsi que
- des additifs et adjuvants usuels,
où
- le rapport de l'EPA ou de ses esters physiologiquement inoffensifs au MTC est entre 1:9 et 3:5,
- la teneur total en graisses est entre 5 et 30 %_{,}
- la teneur en émulsifiant est entre 5 et 12 % par rapport à la teneur en graisses, et
- la teneur en huiles végétales donnant des acides gras en oméga-6 et comprise entre 0 et 30 % (par rapport à la teneur en graisses).

2. Emulsion grasse selon la revendication 1, caractérisée en ce qu'on peut obtenir cette huile de poisson par transformation de poissons d'eau froide, par exemple de maquereaux, de sardines, de harengs ou de saumons.

3. Emulsion grasse selon la revendication 1 ou 2, caractérisée en ce que l'huile de poisson est un concentré d'huile de poisson très purifié, ayant une teneur en EPA de 20 à 40 % et de préférence d'au moins 26 % (par rapport aux esters méthyliques d'acide gras du concentré d'huile de poisson).

4. Emulsion grasse selon les revendications 1 à 3, caractérisée en ce que les triglycérides à longueur moyenne de chaîne contiennent au moins 90 % de glycérides de l'acide caprylique (C₈) et de l'acide caprique (C₁₀).

5. Emulsion grasse selon les revendications 1 à 4, caractérisée en ce que l'émulsifiant utilisé est un phospholipide d'origine animale ou végétale, provenant en particulier du jaune de l'oeuf de poule ou du soja.

6. Emulsion grasse selon les revendications 1 à 5, caractérisée en qu'elle contient d'autres adjuvants d'émulsification, tels que des sels de sodium d'acides gras à longue chaîne (de préférence de 0,005 à 0,1 % en poids par rapport à l'émulsion totale) et/ou du cholestérol ou un ester de cholestérol (de préférence en une quantité de 0,005 à 0,1 % en poids par rapport à l'émulsion totale).

7. Emulsion grasse selon les revendications 1 à 6, caractérisée en qu'on utilise comme huile végétale donnant des acides gras en oméga-6 de l'huile de carthame et/ou de soja.

8. Emulsion grasse selon les revendications 1 à 7, caractérisée en qu'elle contient pour 100 g de graisse de 10 à 1.000 mg d'alpha-tocophérol ou d'un ester de l'alpha-tocophérol.

9. Emulsion grasse selon les revendications 1 à 8, caractérisée en ce qu'elle a un pH compris dans l'intervalle de 6 à 9.

10. Emulsion grasse selon les revendications 1 à 9, destinée à être utilisée dans le métabolisme après agression, en particulier après des opérations et des traumatismes multiples tels que des brûlures, des infections, et en cas d'insuffisance pulmonaire imminente et manifeste.

11. Emulsion grasse selon les revendications 1 à 9, destinée à être utilisée dans les maladies inflammatoires chroniques, notamment dans les maladies inflammatoires chroniques de l'intestin.

12. Emulsion grasse selon les revendications 1 à 9, destinée à être utilisée en néonatalogie et en pédiatrie.

13. Procédé de préparation d'une émulsion grasse isotonique destinée à une administration parentérale, contenant des acides gras en oméga-3, notamment l'acide éicosapentaénoïque (EPA) ou leurs sels physiologiquement inoffensifs, en tant que constituants de la phase grasse, caractérisé en qu'on obtient, par mélange des constituants, cette émulsion grasse qui contient
- ces acides gras en oméga-3 ou ces esters sous forme pure ou en tant que constituants d'huiles de poisson,
- de triglycérides à chaîne de moyenne longueur (MCT),
- au moins un émulsifiant physiologiquement inoffensif,
- éventuellement au moins une huile végétale donnant des acides gras en oméga-6,
- de l'alpha-tocophérol ou un ester d'alpha-tocophérol physiologiquement inoffensif, ainsi que
- des additifs et adjuvants usuels,
où
- le rapport de l'EPA ou de ses esters physiologiquement inoffensifs au MTC est entre 1:9 et 3:5,
- la teneur totale en graisses est entre 5 et 30 %,
- la teneur en émulsifiant est entre 5 et 12 % par rapport à la teneur en graisses, et
- la teneur en huiles végétales donnant des acides gras en oméga-6 est comprise entre 0 et 30 % (par rapport à la teneur en graisses).

14. Procédé selon la revendication 13, caractérisé en ce qu'on obtient l'huile de poisson qui y est utilisée par transformation de poissons d'eau froide, par exemple de maquereaux, de sardines, de harengs ou de saumons.

15. Procédé selon la revendication 13 ou 14, caractérisé en ce qu'on utilise comme huile de poisson un concentré d'huile de poisson très purifié, ayant une teneur en EPA de 20 à 40 % et de préférence d'au moins 26 % (par rapport à l'ester méthylique d'acide gras du concentré d'huile de poisson).

16. Procédé selon les revendications 13 à 15, caractérisé en ce qu'on utilise comme triglycérides à longueur moyenne de chaîne des substances contenant au moins 90 % de glycérides de l'acide caprylique (C₈) et de l'acide caprique (C₁₀).

17. Procédé selon les revendications 13 à 16, caractérisé en qu'on utilise comme émulsifiant un phospholipide d'origine animale ou végétale, provenant en particulier du jaune de l'oeuf de poule ou du soja.

18. Procédé selon les revendications 13 à 17, caractérisé en ce qu'on utilise comme autres adjuvants d'émulsification des sels de sodium d'acides gras à longue chaîne (de préférence de 0,005 à 0,1 % en poids par rapport à l'émulsion totale) et/ou du cholestérol ou un ester de cholestérol (de préférence en une quantité de 0,005 à 0,1 % en poids par rapport à l'émulsion totale).

19. Procédé selon les revendications 13 à 18, caractérisé en ce qu'on utilise comme huile végétale donnant des acides gras en oméga-6 de l'huile de carthame et/ou de l'huile de soja.

20. Procédé selon les revendications 13 à 19, caractérisé en qu'on utilise pour 100 g de graisse 10 à 100 mg d'alpha-tocophérol ou d'un ester de l'alpha-tocophérol.

21. Procédé selon les revendications 13 à 20, caractérisé en ce qu'on ajuste le pH de l'émulsion grasse dans l'intervalle de 6 à 9.

22. Procédé selon les revendications 13 à 21, pour utilisation de l'émulsion grasse qui y est obtenue dans le métabolisme après agression, en particulier après des opérations et des traumatismes multiples tels que des brûlures, des infections, et en cas d'une insuffisance pulmonaire imminente et manifeste.

23. Procédé selon les revendications 13 à 21, pour utilisation de l'émulsion grasse qui y est obtenue, dans les maladies inflammatoires chroniques, notamment dans les maladies inflammatoires chroniques de l'intestin.

24. Procédé selon les revendications 13 à 21, pour utilisation de l'émulsion grasse qui y est obtenue, en néonatalogie et en pédiatrie.

## Revendications (Revendications pour l'(les) Etat(s) contractant(s) suivant(s): ES, GR)

1. Procédé de préparation d'une émulsion grasse isotonique destinée à une administration parentérale, contenant des acides gras en oméga-3, notamment l'acide éicosapentaénoïque (EPA) ou leurs sels physiologiquement inoffensifs, en tant que constituants de la phase grasse, caractérisé en ce qu'on obtient par mélange des constituants cette émulsion grasse, qui contient :
- ces acides gras en oméga-3 ou ces esters sous forme pure ou en tant que constituant d'huiles de poisson,
- de triglycérides à chaîne de moyenne longueur (MCT),
- au moins un émulsifiant physiologiquement inoffensif,
- éventuellement au moins une huile végétale donnant des acides gras en oméga-6,
- de l'alpha-tocophérol ou un ester d'alpha-tocophérol physiologiquement inoffensif, ainsi que
- des additifs et adjuvants usuels,
où
- le rapport de l'EPA ou de ses esters physiologiquement inoffensifs au MTC est entre 1:9 et 3:5,
- la teneur totale en graisses est entre 5 et 30 %,
- la teneur en émulsifiant est entre 5 et 12 % (par rapport à la teneur en graisses) et
- la teneur en huiles végétales donnant des acides gras en oméga-6 est comprise entre 0 et 30 % (par rapport à la teneur en graisses).

2. Procédé selon la revendication 1, caractérisé en ce qu'on obtient l'huile de poisson qui y est utilisée par transformation de poissons d'eau froide, par exemple de maquereaux, de sardines, de harengs ou de saumons.

3. Procédé selon la revendication 1 ou 2, caractérisé en ce qu'on utilise comme huile de poisson un concentré d'huile de poisson très purifié, ayant une teneur en EPA de 20 à 40 % et de préférence d'au moins 26 % (par rapport aux esters méthyliques d'acide gras du concentré d'huile de poisson).

4. Procédé selon les revendications 1 à 3, caractérisé en ce qu'on utilise comme triglycérides à longueur moyenne de chaîne des substances contenant au moins 90 % de glycérides de l'acide caprylique (C₈) et de l'acide caprique (C₁₀).

5. Procédé selon les revendications 1 à 4, caractérisé en ce qu'on utilise comme émulsifiant un phospholipide d'origine animale ou végétale, provenant en particulier du jaune de l'oeuf de poule ou du soja.

6. Procédé selon les revendications 1 à 5, caractérisé en ce qu'on utilise comme autres adjuvants d'émulsification les sels de sodium d'acides gras à longue chaîne (de préférence de 0,005 à 0,1 % en poids par rapport à l'émulsion totale) et/ou du cholestérol ou un ester de cholestérol (de préférence en une quantité de 0,005 à 0,1 % en poids par rapport à l'émulsion totale).

7. Procédé selon les revendications 1 à 6, caractérisé en qu'on utilise comme huile végétale donnant des acides gras en oméga-6 de l'huile de carthame et/ou de soja.

8. Procédé selon les revendications 1 à 7, caractérisé en ce qu'on utilise pour 100 g de graisse 10 à 100 mg d'alpha-tocophérol ou d'un ester de l'apha-tocophérol.

9. Procédé selon les revendications 1 à 8, caractérisé en ce qu'on ajuste le pH de l'émulsion grasse dans l'intervalle de 6 à 9.

10. Procédé selon les revendications 1 à 9, pour utilisation de l'émulsion grasse qui y est obtenue dans le métabolisme après agression, en particulier après des opérations et des traumatismes multiples tels que des brûlures, des infections, et en cas d'une insuffisance pulmonaire imminente et manifeste.

11. Procédé selon les revendications 1 à 10, pour utilisation de l'émulsion grasse qui y est obtenue, dans les maladies inflammatoires chroniques, notamment dans les maladies inflammatoires chroniques de l'intestin.

12. Procédé selon les revendications 1 à 10, pour utilisation de l'émulsion grasse qui y est obtenue, en néonatalogie et en pédiatrie.
